# EUROPEAN PATENT SPECIFICATION

(11) **EP 3 269 420 B1**
(45) Date of publication and mention of the grant of the patent: **19.02.2020**
(21) Application number: 15801975.2
(22) Date of filing: 19.03.2015
(51) Int. Cl.: A61N 1/32

(54) **ELECTRICAL MUSCLE STIMULATION DEVICE**
VORRICHTUNG ZUR ELEKTRISCHEN MUSKELSTIMULATION
DISPOSITIF DE STIMULATION ÉLECTRIQUE DES MUSCLES

(30) Priority: 06.03.2015 JP 2015045283
(43) Date of publication of application: 17.01.2018
(73) Proprietor: MTG Co., Ltd., Aichi 453-0041 (JP)
(72) Inventor: MATSUSHITA Tsuyoshi, Nagoya-shi Aichi 453-0041 (JP)
(74) Representative: Kramer Barske Schmidtchen Patentanwälte PartG mbB
(86) International application number: PCT/JP2015/058230
(87) International publication number: WO 2016/143145

(56) References cited:
- EP-A1- 0 641 230
- WO-A1-2013/171445
- AU-A1- 2014 200 220
- GB-A- 2 438 589
- JP-A- H08 501 946
- JP-A- 2001 293 097
- JP-A- 2001 293 097
- JP-A- 2003 019 216
- JP-A- 2006 271 689
- US-A1- 2015 012 066
- US-B1- 8 620 438
- US-B2- 6 526 319

## Description

### Technical Field

The present invention relates to a muscle electrostimulation device.

### Background Art

Conventionally, it has been widely known that the flow of electric current through muscle fibers causes muscle contraction. In particular, this knowledge has been utilized for the sake of building muscles in medical and sports fields. More specifically, a muscle stimulating method of supplying electricity through electrodes attached on a human body to contract and relax muscles on the basis of an electric signal has been used. It is known that low frequency signals are specifically effective as electric signals for contracting muscles, because muscles tend not to contract as the frequency of the electric signal increases.

However, a low frequency electric signal tends to cause pain owing to adverse effects of the electric resistance on human skin and the like. On the contrary, a high frequency electric signal tends to reduce the adverse effects of the electric resistance and the like, thereby achieving characteristics hardly causing pain.

As a muscle stimulation device for stimulating muscles using an electric signal, Patent Document 1 discloses a technique that includes a main body containing a power source, and a pair of electrodes elongated from the main body, attaches the pair of electrodes on a human body, and causes an electric pulse to flow through the human body to stimulate muscles.

### Prior Art Document

### Patent Document

Patent Document 1: JP-B-3158303
EP 0 641 230 A1 discloses electro-stimulation for treatment of incontinence and other neuro-muscular disorders. A portable electrical stimulation apparatus comprises one or more electrodes, a signal generator, one or more conductive leads for connecting the signal generator to the electrodes, and a power supply. WO 2013/171445 A1 discloses an apparatus for treatment of an animal or human which comprises a pulse delivery system for generating a series of electrical current pulses having a pre-programmed waveform.

### Summary of the Invention

### Problem to be Solved by the Invention

However, according to the configuration disclosed in Patent Document 1, a user sometimes feels pain on his/her skin owing to the electric pulse. There is a room for improvement in sensitivity during using the muscle stimulation device. On the other hand, only reduction in the voltage of the electric pulse or simple increase in frequency in order to reduce the pain of the user cannot efficiently stimulate muscles.

The present invention has been made in view of such a background, and has an object to provide a muscle electrostimulation device capable of improving sensitivity in use and efficiently stimulating muscles.

### Means for Solving the Problem

This object is achieved by a muscle electrostimulation device in accordance with claim 1. Further developments of the invention are recited in the dependent claims.

### Effects of the Invention

According to the muscle electrostimulation device, in the burst wave for forming electrostimulation, the rectangular wave pulse signals are output, with the output stop time being inserted between the rectangular wave pulse signals in the pulse group output period. Thus, in the pulse group output period, the rectangular wave pulse signal is in a state of being divided into multiple pieces. Accordingly, in comparison with the case of continuously outputting the rectangular wave pulse signals without division over the pulse group output period, each of individual pulse widths of the rectangular wave pulse signals can be small while maintaining the total output time of rectangular wave pulse signals to be the same. As a result, the pain of the user can be alleviated while maintaining electrostimulation output from the muscle electrostimulation device and flowing into muscles or nerves connected to muscles. Accordingly, the sensitivity during use of the muscle electrostimulation device can be improved.

In comparison with a burst wave having a pulse outputting period of continuously outputting pulses for the same period as the pulse group output period in the burst wave, the burst wave described above allows multiple rectangular wave pulse signals to be output, with the output stop time being inserted between the rectangular wave pulse signals in the pulse group output period. However, both the burst waves have the same period of outputting pulses. Accordingly, the burst waves having the pulse group output periods, with the output stop times being inserted between the rectangular wave pulse signals can achieve sensitivity similar to the sensitivity of the burst waves having pulse outputting periods, with no output stop time being inserted between the rectangular wave pulse signals.

In the pulse group output period, the rectangular wave pulse signals are output, with the output stop times being inserted between the rectangular wave pulse signals. Thus, the duration of the pulse group output period is the total of the pulse widths of the rectangular wave pulse signals and all the output stop times. Accordingly, in comparison with the case of continuing outputting the rectangular wave pulse signals over the duration of the pulse group output period, the actual pulse signal output time is reduced by the output stop times while maintaining the duration of the pulse group output period to be the same. The power consumption can therefore be reduced. This reduction allows driving even by a power source having a small capacity, thereby contributing to reduction in size of the device.

The burst wave forming electrostimulation includes the pulse group output period and any of the pulse group output suspension periods. The durations of the pulse group output suspension periods are longer than the output stop times in the pulse group output period. Since the burst waves include such pulse group output suspension periods, the frequencies of the burst waves can be set to a desired value by only changing the duration of each of the pulse group output suspension periods to a predetermined length without changing the pulse group output period. This setting facilitates control for outputting electrostimulation including the burst wave having a frequency suitable for contracting and relaxing muscles, and can efficiently stimulate the muscles.

The repetitive burst waves include at least one rectangular wave pulse signal having positive polarity, and at least one rectangular wave pulse signal having negative polarity. That is, one burst wave may include both the rectangular wave pulse signal having positive polarity, and the rectangular wave pulse signal having negative polarity. Alternatively, in multiple burst waves, a first burst wave may include at least one rectangular wave pulse signal having positive polarity, and a second burst wave different from the first burst wave may include at least one rectangular wave pulse signal having negative polarity. In both the cases, deviation in electric charge can be reduced, thereby allowing the pain of the user to be alleviated. As a result, the sensitivity and the usefulness during use of the muscle electrostimulation device can be improved.

As described above, the present invention can provide a muscle electrostimulation device capable of improving sensitivity in use and efficiently stimulating muscles.

### Brief Description of the Drawings

[Figure 1] Figure 1 is a front view of a muscle electrostimulation device of Embodiment 1.
[Figure 2] Figure 2 is a rear view of the muscle electrostimulation device of Embodiment 1.
[Figure 3] Figure 3 is a side view of the muscle electrostimulation device of Embodiment 1.
[Figure 4] Figure 4 is a partially enlarged sectional view taken along line IV-IV of Figure 1.
[Figure 5] Figure 5 is a schematic diagram illustrating a usage mode of the muscle electrostimulation device of Embodiment 1.
[Figure 6] Figure 6 is a block diagram showing a configuration of the muscle electrostimulation device of Embodiment 1.
[Figure 7] Figure 7 is a diagram showing basic waveforms stored in the muscle electrostimulation device of Embodiment 1.
[Figure 8] Figure 8 is a diagram showing burst waves output by the muscle electrostimulation device of Embodiment 1.
[Figure 9] Figure 9 is a diagram showing variation in voltage output by the muscle electrostimulation device of Embodiment 1.
[Figure 10] Figure 10 is a flowchart illustrating a main operation of the muscle electrostimulation device of Embodiment 1.
[Figure 11] Figure 11 is a flowchart illustrating a first interrupt process of the muscle electrostimulation device of Embodiment 1.
[Figure 12] Figure 12 is a flowchart illustrating a second interrupt process of the muscle electrostimulation device of Embodiment 1.
[Figure 13] Figure 13 is a flowchart illustrating a third interrupt process of the muscle electrostimulation device of Embodiment 1.
[Figure 14] Figure 14 is a front view of a muscle electrostimulation device of Variation 2.
[Figure 15] Figure 15 is a rear view of the muscle electrostimulation device of Variation 2.
[Figure 16] Figure 16 is a block diagram showing a configuration of a muscle electrostimulation device of Variation 3.

### Mode for Carrying out the Invention

The muscle electrostimulation device outputs electrostimulation including burst waves. The electrostimulation output by the muscle electrostimulation device flows into muscles or nerves connected to muscles to stimulate the muscles.

The rectangular wave pulse signal having positive polarity and the rectangular wave pulse signal having negative polarity may be included in the same pulse group output period. In this case, deviation in electric charge can be easily eliminated in one burst wave, thereby allowing the pain of the user to be further alleviated. As a result, the sensitivity and the usefulness during use of the muscle electrostimulation device can be further improved.

The repeatedly output burst wave may include: a first burst wave; and a second burst wave including a second pulse group output period for outputting the multiple rectangular wave pulse signals having polarity inverted from the polarity of the multiple rectangular wave pulse signals output in the first pulse group output period of the first burst wave. In this case, when deviation in electric charge occurs in the first burst wave, the deviation in electric charge can be securely eliminated by the second burst wave. Accordingly, deviation in electric charge can be reduced over the entire repeatedly output burst wave, thereby allowing the pain of the user to be alleviated. As a result, the sensitivity and the usefulness during use of the muscle electrostimulation device can be further improved. Furthermore, the second pulse group output period can be achieved only by inverting the polarity (inverting the potential) of the multiple rectangular wave pulse signals output in the first pulse group output period. Accordingly, the control load can be reduced in comparison with the case of separately controlling the polarities of the individual rectangular wave pulse signals in each burst wave. This feature is analogous to the case of a burst wave including multiple pulse groups.

The duration of the pulse group output suspension period may be longer than the duration of the pulse group output period. In this case, the pulse group output suspension period can sufficiently provide the interval between the pulse group output periods repeatedly output in the burst wave. Accordingly, the user can easily recognize the multiple rectangular wave pulse signals in the pulse group output period as one electrostimulation. As a result, a low-frequency burst wave can be easily output from the high-frequency rectangular wave pulse signal, thereby allowing electrostimulation suitable for stimulating muscles to be output.

The device may include: a burst wave pattern memory preliminarily storing multiple burst wave patterns having different frequencies from each other due to difference in duration of the pulse group output suspension periods while the duration of the pulse group output period being the same; and a frequency setting unit for setting the frequency of the burst wave for the electrostimulation by selecting any of the burst wave patterns stored in the burst wave pattern memory. In this case, the burst wave pattern memory preliminarily stores the multiple burst wave patterns having a predetermined frequency. Accordingly, changing of the frequency of the burst wave only requires the frequency setting unit to select a predetermined pattern from among the burst waves stored in the burst wave pattern memory. This requirement facilitates changing of the frequency of burst wave. Accordingly, the muscle electrostimulation device suitable for efficiently stimulating muscles can be achieved.

The device may include: a frequency selector for selecting the frequency of the burst wave; a suspension period duration calculator for calculating the duration of the pulse group output suspension period on the basis of the frequency selected by the frequency selector; and a suspension period duration setting unit for setting the duration of the pulse group output suspension period on the basis of the duration calculated by the suspension period duration calculator. In this case, the frequency selector can set the frequency of the burst wave to a desired frequency. Accordingly, the frequency of the burst wave is appropriately set on the basis of the preferences of users (the strength of contraction, and the interval between contraction and relaxation), thereby allowing the muscle electrostimulation device to further efficiently stimulate muscles in conformity with the individual users. Selection of the frequency at the frequency selector can be performed automatically or manually by the user.

The pulse width of the rectangular wave pulse signal in the burst wave and the output stop times may be constant. In this case, electrostimulation to be applied to muscles can be easily changed on the basis of the frequency of the burst wave. Accordingly, adjustment of the electrostimulation by the frequency of the burst wave becomes easier, thereby facilitating output of electrostimulation suitable for effectively stimulating the muscles.

The device may include: a main body; multiple electrode units for outputting the electrostimulation; a power source for supplying power to the electrode units; a controller for controlling power supply by the power source; and an operation unit configured to be capable of changing the control mode of the controller. The power source may be embedded in the main body. In this case, electric power to be supplied to the electrode unit is not required to be prepared externally. Accordingly, the device can be easily used even outdoors and in places away from home where it is difficult to secure a power source. Furthermore, this case does not require a cord and the like for connection to the power source. Accordingly, the usability is improved, and the portability is excellent.

The electrode unit may include multiple electrodes and leads for electrically connecting the electrodes to the power source via the controller, on the sheet-shaped substrate extending from the main body. In this case, the electrode unit is formed on the sheet-shaped substrate extending from the main body, thereby allowing the main body and the electrode unit to be integrally configured. This configuration does not require a cord and the like for connecting the main body to the electrode unit. Accordingly, the usability is improved, and the portability is excellent.

The electrode unit may include at least three electrodes. As described above, in the pulse group output period, with output stop times being inserted between multiple rectangular pulses, thereby reducing the power consumption. Accordingly, even with the configuration including at least three electrodes, the power hardly lacks, thereby enabling sufficient electrostimulation to be applied. Therefore, electrostimulation can be applied to a wide range of muscles, thereby allowing muscles to be efficiently stimulated.

The power source may include a replaceable battery. In this case, the power is supplied only by replacing the battery, thereby facilitating long-time use exceeding the capacity of the battery. This configuration does not require embedding a battery having an excessively large capacity, thereby allowing the device to be reduced in size.

The battery may be a coin battery or a button battery. In this case, the battery has a small size, thereby contributing to reduction in size of the muscle electrostimulation device. The reduction in size of the muscle electrostimulation device can, in turn, reduce the weight. Accordingly, the electrodes become resistant to falling and dropping off the body of the user, thereby improving the usability and portability. Furthermore, the battery is thin, thereby contributing to reduction in thickness of the muscle electrostimulation device. The thinly shaped muscle electrostimulation device allows the user to wear clothes on muscle electrostimulation device, with the device being left to be worn. Accordingly, the device can be used during commuting to the workplace or school, doing housework and working, and in other various situations. The coin battery or the button battery has higher operating voltage and more stable discharge characteristics than the other dry batteries. Accordingly, the muscle electrostimulation device can be stably operated for a relatively long time.

The battery may have a nominal voltage ranging from 3.0 to 5.0 V. In this case, for many of the electronic components included in the device, drive voltages are defined in such a voltage range. Accordingly, a step-down circuit or a booster circuit is not required to be separately provided to drive these electronic components, thereby contributing to reduction in size. Since the electrostimulation output from the electrodes typically requires a voltage higher than the nominal voltage of the battery, the configuration does not necessarily negate intervention of a booster device for output.

### Embodiments

### (Embodiment 1)

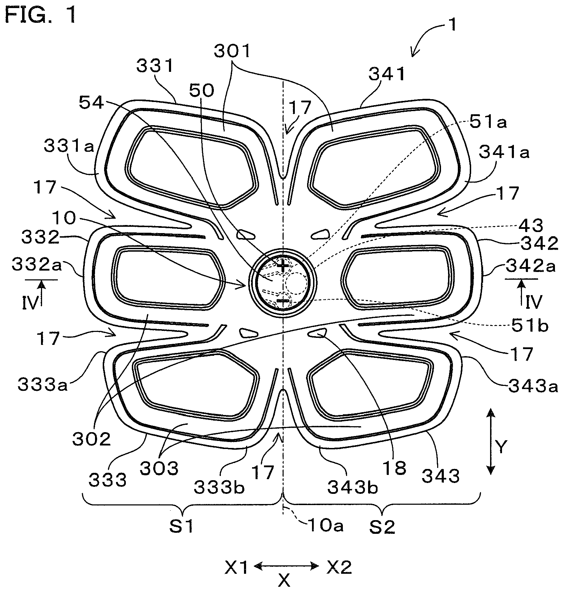

A muscle electrostimulation device according to an embodiment is described with reference to Figures 1 to 13.

The muscle electrostimulation device 1 of this embodiment is configured to provide electrostimulation to muscles.

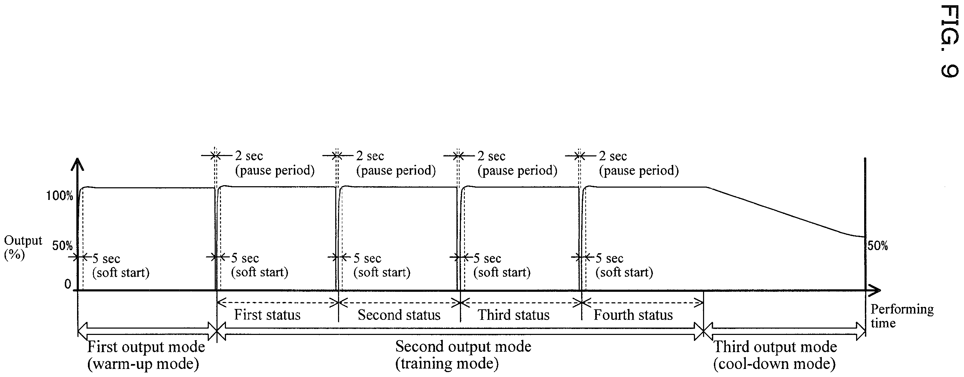

As shown in Figure 9, the electrostimulation includes any of burst waves (B1 to B5) having a pulse group output period P and any of pulse group output suspension periods R1 to R5, the burst waves being repeatedly output.

In the pulse group output period P, multiple rectangular wave pulse signals S1 to S5 are output, each of output stop times N1 to N5 being inserted between the signals.

Each of the pulse group output suspension periods R1 to R5 is longer than each of the output stop times N1 to N5. During the periods, output of the pulse signals is stopped.

The repetitive burst waves (B1 to B5) include rectangular wave pulse signals S1, S3 and S5 having positive polarity, and rectangular wave pulse signals S2 and S4 having negative polarity.

The muscle electrostimulation device 1 is hereinafter described in detail.

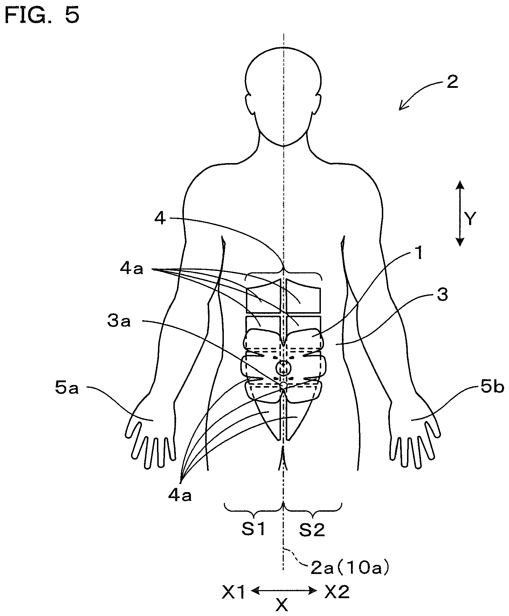

As shown in Figure 5, the muscle electrostimulation device 1 of this embodiment is used while being attached to the abdomen 3 of a person 2. In this embodiment, the longitudinal direction of the height of the person 2 is referred to as a height direction Y. Facing the front side of the person 2, a right hand side 5a of the person 2 with respect to the central axis 2a of the person 2 passing through the umbilicus 3a in parallel to the height direction Y is referred to as a right direction X1. Facing the front side of the person 2, a left hand side 5b of the person 2 with respect to the central axis 2a is referred to as a left direction X2. The right direction X1 and the left direction X2 are integrally referred to as a lateral direction X.

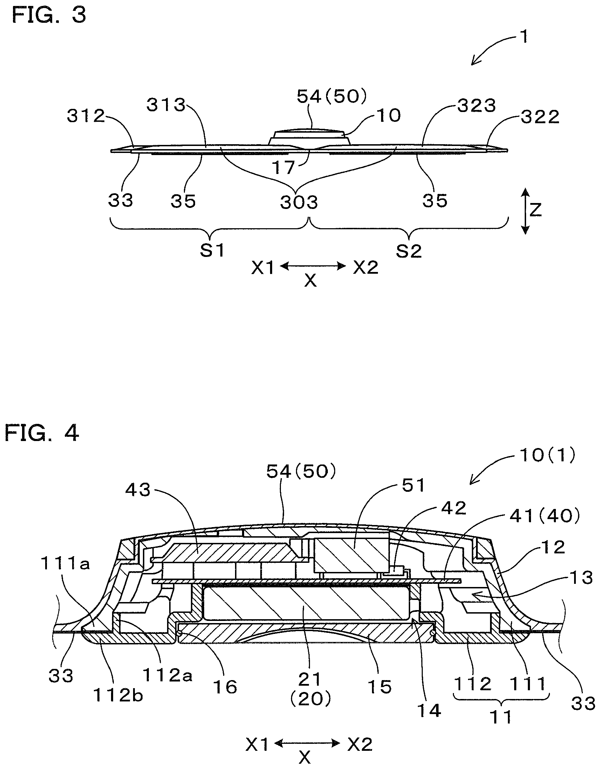

As shown in Figure 1, a main body 10 is provided at the center of the muscle electrostimulation device 1. As shown in Figures 1 and 3, the main body 10 substantially has a disc shape. As shown in Figure 4, the main body 10 includes a case 11 for storing a power source 20 and a controller 40, described later, and a shell forming body 12 attached to the case 11 to form a shell of the muscle electrostimulation device 1. The case 11 is made of ABS. The shell forming body 12 is made of silicone. The case 11 includes a concave first case 111, and a second case 112 attached to the first case 111 to form a container 13, together with the first case 111, for storing the controller 40. A rib 112a provided to stand along the periphery of the second case 112 is fitted with the inside of a peripheral portion 111a of the first case 111, thus joining the second case 112 to the first case 111.

As shown in Figure 1, a first cantilever 51a and a second cantilever 51b are formed at the first case 111; each cantilever forms a part of an after-mentioned operation unit 50. The first cantilever 51a and the second cantilever 51b are formed to have a cantilever beam shape by removing a part of a wall of the first case 111. The first cantilever 51a and the second cantilever 51b are arranged from the upper side to the lower side in the height direction Y in this order.

As shown in Figure 4, the shell forming body 12 is attached to the first case 111 on a side not facing the second case 112. As shown in Figure 1, the shell forming body 12 covers both the cantilevers 51a and 51b. On the shell forming body 12, a symbol "+" is formed to protrude immediately above the first cantilever 51a, and a symbol "-" is formed to protrude immediately above the second cantilever 51b, thereby forming an operation surface 54 forming a part of the after-mentioned operation unit 50. The arrangement of the cantilevers disposes the "+" on an upper side and the "-" on a lower side in the height direction Y. This arrangement allows a user to perform ergonomically easy operation.

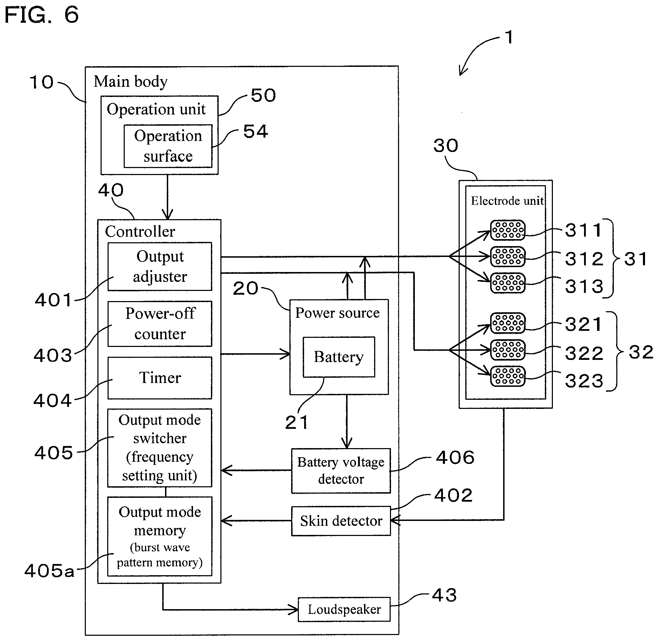

As shown in Figure 4, the container 13 formed between the first case 111 and the second case 112 contains a control board 41 forming the controller 40 (see Figure 6). The control board 41 is a printed board. The control board 41 is provided with a wiring pattern, electronic components 42 and the like, not shown, to form a control circuit. A small-sized surface-mounted loudspeaker 43 is electrically connected to the control board 41. Each of the drive voltages for the electronic component 42 and the loudspeaker 43 is 3.0 V. Although not shown, a booster circuit for increasing the output voltage of the battery 21 is mounted on the control board 41. Accordingly, the power of the battery 21 is increased to a predetermined voltage (e.g., 40 V), and supplied to an electrode unit 30.

Although not shown, the container 13 also contains switch mechanisms forming the operation unit 50. The switch mechanisms are tactile switches, and include switch sections that can be pressed. The switch mechanisms are electrically connected to the controller 40. The switch mechanisms are arranged immediate below the first cantilever 51a and the second cantilever 51b (see Figure 1) formed on the first case 111. Accordingly, a press on the first cantilever 51a from the outside via the operation surface 54 of the shell forming body 12 covering the first case 111 bends the cantilever-beam-shaped first cantilever 51a, thereby pressing the switch section of the switch mechanism. Release of the press on the operation surface 54 allows the first cantilever 51a to return to the original position owing to the resilience of the cantilever-beam-shaped first cantilever 51a. Likewise, the second cantilever 51b is subjected to pressing and releasing of the pressing.

As shown in Figure 4, at the second case 112, a battery holder 14 for holding the battery 21 configuring the power source 20 is formed. The power source 20 is thus embedded in the main body 10. The battery 21 is replaceable. In this embodiment, a small and thin coin battery (lithium-ion battery CR2032, nominal voltage 3.0 V) is adopted as the battery 21. Instead of the battery 21, a battery having a nominal voltage of 3.0 to 5.0 V may be adopted.

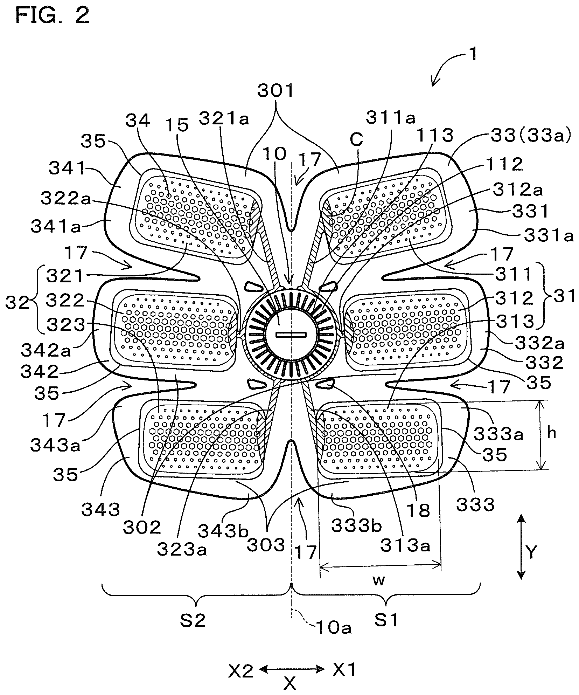

A lid 15 for preventing the battery 21 from dropping off is detachably attached to the battery holder 14 holding the battery 21. The lid 15 has a disc shape one-size-larger than the battery 21. An O-ring 16 for sealing the lid 15 and the second case 112 is fitted around the periphery of the lid 15. The battery 21 is electrically connected to the controller 40 via a lead, not shown. As shown in Figure 2, a plurality of linear grooves 113 radially extending from the periphery of the lid 15 is formed on the second case 112 at regular intervals.

As shown in Figure 4, a flange 112b protruding outward from the rib 112a is formed at the second case 112. A sheet-shaped substrate 33 is sandwiched by the flange 112b and the peripheral portion 111a of the first case 111 via double-sided waterproof seal, not shown. The substrate 33 is made of PET. As shown in Figure 2, the substrate 33 extends over an entire surface (rear surface 33a) opposite to a surface (front surface) on a side of the shell forming body 12 in the muscle electrostimulation device 1. As shown in Figures 1 and 3, the front surface of the substrate 33 is covered with the shell forming body 12 extending from the main body 10. The substrate 33 and the shell forming body 12 are joined to each other by adhesive tape and silicone adhesion treating agent, not shown, manufactured by 3M Company.

As shown in Figures 2 and 6, the electrode unit 30 includes a first electrode group 31 and a second electrode group 32. As shown in Figure 5, the first electrode group 31 extends from the main body 10 so as to be disposed right direction X1 of the person 2 with respect to the center line 10a when attached to the abdomen 3. As shown in Figure 5, the second electrode group 32 extends from the main body 10 so as to be disposed left direction X2 of the person 2 with respect to the center line 10a when attached to the abdomen 3. The first electrode group 31 includes right electrodes 311 to 313. The second electrode group 32 includes left electrodes 321 to 323.

Each of the electrodes 311 to 313 and 321 to 323 is formed to have a substantially rectangular shape with rounded corners. The longitudinal direction (e.g., a direction indicated by a symbol w on the third right electrode 313) of each of the electrodes 311 to 313 and 321 to 323 is substantially along the lateral direction X. In this embodiment, each of the electrodes 311 to 313 and 321 to 323 has the same shape. The shape of each of the electrodes 311 to 313 and 321 to 323 has, for example, h/w of 0.40 to 0.95, preferably, 0.50 to 0.80, where w is the length in the longitudinal direction and h is the length in the short transverse direction. In this embodiment, h/w is 0.55.

As shown in Figure 2, in each of the electrodes 311 to 313 and 321 to 323, hexagonal-shaped portions 34 that form no electrode and have a predetermined size are formed at regular intervals. Leads 311a, 312a and 313a for electric connection to the power source 20 via the controller 40 are formed at the respective right electrodes 311, 312 and 313 so as to be drawn from the main body 10. Likewise, leads 321a, 322a and 323a for electric connection to the controller 40 are formed at the respective left electrodes 321, 322 and 323 so as to be drawn from the main body 10. The leads 311a to 313a and 321a to 323a are silicone-coated to prevent electric conduction to the outside. In the electrodes 311 to 313 and 321 to 323, portions connected to the leads 311a to 313a and 321a to 323a and adjacent areas (hatched areas indicated by a symbol C in Figure 2) are also silicone-coated to prevent electric conduction to the outside. The right electrodes 311 to 313 are connected in parallel to each other. The left electrodes 321 to 323 are also connected in parallel to each other.

As shown in Figure 2, the electrode unit 30 is formed on the rear surface 33a of the substrate 33. Thus, the electrode unit 30 is formed integrally with the main body 10. The electrode unit 30 may be formed so as to be embedded in the substrate 33. In this embodiment, the electrode unit 30 is formed by printing electrically conductive ink containing silver paste on the rear surface 33a of the substrate 33. The first electrode group 31 and the second electrode group 32 include at least four, in total, electrodes 311 to 313 and 321 to 323. In this embodiment, the first electrode group 31 and the second electrode group 32 include the same numbers of electrodes 311 to 313 and 321 to 323. In each group, the number of electrodes is three. That is, the first electrode group 31 includes the first right electrode 311, the second right electrode 312, and the third right electrode 313. The second electrode group 32 includes the first left electrode 321, the second left electrode 322, and the third left electrode 323. On the substrate 33, portions bearing the first right electrode 311, the second right electrode 312 and the third right electrode 313 are referred to as a first right base part 331, a second right base part 332, and a third right base part 333, respectively. Portions bearing the first left electrode 321, the second left electrode 322, and the third left electrode 323 are referred to as a first left base part 341, a second left base part 342, and a third left base part 343, respectively.

A gel pad 35 ("ST-gel" manufactured by Sekisui Plastics Co., Ltd., model No. SR-RA240/100) is pasted on each of the electrodes 311 to 313 and 321 to 323. The gel pad 35 is electroconductive. The electrodes 311 to 313 and 321 to 323 can energize the abdomen 3 (see Figure 5) via the gel pad 35. The gel pad 35 is strongly adhesive. Accordingly, the muscle electrostimulation device 1 can be attached to the abdomen 3 via the gel pad 35.

As shown in Figure 2, the gel pads 35 are one-size-larger than the electrodes 311 to 313 and 321 to 323, and separately cover the respective electrodes 311 to 313 and 321 to 323. The gel pads 35 are replaceable. Accordingly, if the gel pads 35 deteriorate in adhesive force, are damaged or become dirty in accordance with usage, these pads can be appropriately replaced. The used gel pads 35 may be replaced with new pads at every predetermined time period (e.g., one month, two months, etc.)

As shown in Figure 2, each of the first right electrode 311, the second right electrode 312, and the third right electrode 313 extends from the main body 10 so as to be disposed right direction X1 of the person 2 (first area S1) with respect to the center line 10a passing through the center of the main body 10 in parallel to the height direction Y of the person 2 (see Figure 5). The first right electrode 311, the second right electrode 312, and the third right electrode 313 are arranged from the upper side to the lower side in the height direction Y in this order.

On the other hand, each of the first left electrode 321, the second left electrode 322, and the third left electrode 323 extends from the main body 10 so as to be disposed left direction X2 of the person 2 (second area S2) with respect to the center line 10a. The first left electrode 321, the second left electrode 322, and the third left electrode 323 are arranged from the upper side to the lower side in the height direction Y in this order.

As shown in Figure 2, the first electrode group 31 and the second electrode group 32 are configured to be disposed line-symmetrical with respect to the center line 10a when attached to the abdomen 3 (see Figure 5). That is, when attached to the abdomen 3, with respect to the center line 10a, the first right electrode 311 and the first left electrode 321 are disposed line-symmetrical, the second right electrode 312 and the second left electrode 322 are line-symmetrical, and the third right electrode 313 and the third left electrode 323 are line-symmetrical.

As shown in Figure 2, when attached to the abdomen 3 (see Figure 5), in the height direction Y, the first electrode group 31 and the second electrode group 32 are configured to include: an upper electrode pair 301 of the first right electrode 311 and the first left electrode 321 disposed uppermost of the first electrode group 31 and the second electrode group 32, respectively; a lower electrode pair 303 of the third right electrode 313 and the third left electrode 323 disposed lowermost; and a central electrode pair 302 of the second right electrode 312 and the second left electrode 322 disposed between the upper electrode pair 301 and the lower electrode pair 303. Thus, the upper electrode pair 301, the central electrode pair 302, and the lower electrode pair 303 are arranged from the upper side to the lower side in the height direction Y in this order.

The central electrode pair 302 projects in the extending direction of the main body 10 (lateral direction X) more than the upper electrode pair 301 and the lower electrode pair 303. That is, when attached to the abdomen 3, the second right electrode 312 included in the central electrode pair 302 projects in the right direction X1 more than the first right electrode 311 included in the upper electrode pair 301, and the third right electrode 313 included in the lower electrode pair 303. Likewise, the second left electrode 322 included in the central electrode pair 302 projects in the left direction X2 more than the first left electrode 321 included in the upper electrode pair 301, and the third left electrode 323 included in the lower electrode pair 303.

As shown in Figure 2, the upper electrode pair 301 arranged inclined so as to have a V-shape deviating upward with being far in the extending direction. As described above, each of the electrodes 311 to 313 and 321 to 323 has the same size. On the other hand, the right base parts 331 to 333 of the substrate 33 of the electrode unit 30 have larger sizes than the respective right electrodes 311 to 313. The left base parts 341 to 343 have larger sizes than the respective left electrodes 321 to 323.

As shown in Figure 2, the upper electrode pair 301 projects in the direction (lateral direction X) extending from the main body 10 more than the lower electrode pair 302. That is, when attached to the abdomen 3, the first right electrode 311 included in the upper electrode pair 301 projects in the right direction X1 more than the third right electrode 313 included in the lower electrode pair 303. Likewise, the first left electrode 321 included in the upper electrode pair 301 projects in the left direction X2 more than the third left electrode 323 included in the lower electrode pair 303.

As shown in Figure 2, a lower peripheral part 331a of the first right base part 331 protrudes in the right direction X1. A lower peripheral part 341a of the first left base part 341 protrudes in the left direction X2.

A central peripheral part 332a of the second right base part 332 slightly protrudes in the right direction X1. A central peripheral part 342a of the second left base part 342 slightly protrudes in the left direction X2.

Furthermore, an upper peripheral part 333a of the third right base part 333 protrudes in the right direction X1. A lower peripheral part 333b of the third right base part 333 protrudes downward (in a downward sense in the Y direction). An upper peripheral part 343a of the third left base part 343 protrudes in the left direction X2. A lower peripheral part 343b of the third left base part 343 protrudes downward.

As shown in Figures 1 and 5, the configuration of the base parts 331 to 333 and 341 to 343 of the substrate 33 as described above allows the muscle electrostimulation device 1 to wrap the rectus abdominis muscle 4 in the abdomen 3 in the lateral direction in view of the muscle electrostimulation device 1 from the front. The electrode arrangement conforms to the compartments 4a of the rectus abdominis muscle 4. It can thus be expected to efficiently stimulate each muscle. Furthermore, the user can imagine the state of shaped abdomen 3 with six pack abdominal muscles by recognizing such a shape. Accordingly, use of the muscle electrostimulation device 1 can exert advantageous effects of image training for achieving the shaped abdomen 3 with six pack abdominal muscles (improvement in athletic effects due to image training is widely known to the public.)

As shown in Figure 2, in the first electrode group 31 and the second electrode group 32, notches 17 cut into the main body 10 are formed between the electrodes 311 to 313 and 321 to 323 adjacent to each other. In this embodiment, the notches 17 are formed at six positions, i.e., between the first right electrode 311 and the second right electrode 312, between the second right electrode 312 and the third right electrode 313, between the third right electrode 313 and the third left electrode 323, between the third left electrode 323 and the second left electrode 322, between the second left electrode 322 and the first left electrode 321, and between the first left electrode 321 and the first right electrode 311. Furthermore, through holes 18 are formed at four positions around the main body 10.

Next, the configuration of the muscle electrostimulation device 1 of this embodiment is described with reference to a block diagram.

As shown in Figure 6, the muscle electrostimulation device 1 includes not only the power source 20, the controller 40 and the operation unit 50, but also a skin detector 402 and a battery voltage detector 406, in the main body 10.

The skin detector 402 detects whether the electrode unit 30 is in contact with skin or not. More specifically, the skin detector 402 is electrically connected to the electrode unit 30 to detect a resistance value between the first electrode group 31 and the second electrode group 32. This detector then compares the detected value with a preset threshold. If the detected value is smaller than the threshold, this detector detects that the first electrode group 31 and the second electrode group 32 are in contact with the skin.

The battery voltage detector 406 detects the voltage of the battery 21 in the power source 20, and determines whether the detected battery voltage V of the battery 21 of the power source 20 is lower than a predetermined threshold Vm or not. In this embodiment, the nominal voltage V of the battery 21 is 3.0 V, and the threshold Vm is 2.1 V.

As shown in Figure 6, the power source 20 includes the battery 21. The controller 40 includes an output adjuster 401, a power-off counter 403, a timer 404, an output mode switcher 405, and an output mode memory 405a. The output adjuster 401 adjusts the output voltage (output level) at the electrode unit 30. In this embodiment, the maximum output voltage is set to 40 V. 100% output voltage is set to be reduced by 2.0 V at every decrease in output level by one. The output level ranges over 15 steps, from a level 1 to a level 15.

The power-off counter 403 measures the elapsed time after receipt of a count start signal. The timer 404 measures the elapsed time after receipt of an output start signal. The output mode switcher 405 switches the output mode of the electrode unit 30 to any of a first output mode, a second output mode and a third output mode, and configures a frequency setting unit for setting the frequency of the burst wave to be output. The output mode memory 405a stores the first output mode, the second output mode, and the third output mode. The first output mode, the second output mode, and the third output mode are preliminarily stored with basic waveforms, i.e. burst wave patterns, having pulse group output suspension periods R1 to R5. The output mode memory 405a configures a burst wave pattern memory. The burst wave pattern memory 405a further stores description of definition of the waveforms of burst waves on a program.

Next, the output modes in the electrode unit 30 are described.

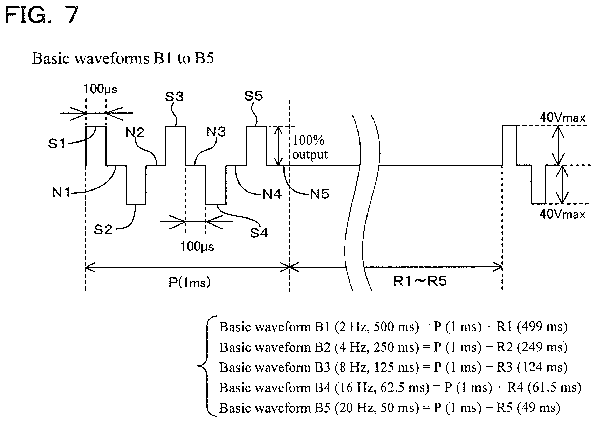

First, the output mode memory 405a, as a duration memory, stores five burst wave patterns (basic waveforms B1 to B5) shown in Figure 7. The basic waveforms B1 to B5 includes the pulse group output period P, and the pulse group output suspension periods R1 to R5, respectively. That is, each of the basic waveforms B1 to B5 include the common pulse group output period P, and further includes any of the pulse group output suspension periods R1 to R5 having different lengths.

In the pulse group output period P, the rectangular wave pulse signals S1 to S5 are output, with the output stop times N1 to N5 being inserted between the rectangular wave pulse signals S1 to S5. In this embodiment, five rectangular wave pulse signals S1 to S5 are output. That is, in the pulse group output period P, the order of execution is the first rectangular wave pulse signal S1, the first output stop time N1, the second rectangular wave pulse signal S2, the second output stop time N2, the third rectangular wave pulse signal S3, the third output stop time N3, the fourth rectangular wave pulse signal S4, the fourth output stop time N4, the fifth rectangular wave pulse signal S5, and the fifth output stop time N5.

In this embodiment, the pulse width and the pulse voltage of each of the rectangular wave pulse signals S1 to S5 are constant. The duration of each of the output stop times N1 to N5 is also constant. In this embodiment, each of the rectangular wave pulse signals S1 to S5 has a pulse width of 100 µs, and a pulse voltage of ±40 V during 100% output. The duration of each of the output stop times N1 to N5 is 100 µs. Accordingly, the duration of the pulse group output period P is 1 ms. The voltage polarities of the rectangular wave pulse signals S1 to S5 are alternately changed according to the output order. That is, the first rectangular wave pulse signal S1, the third rectangular wave pulse signal S3 and the fifth rectangular wave pulse signal S5 are output as the rectangular wave pulse signals having positive polarity. The second rectangular wave pulse signal S2 and the fourth rectangular wave pulse signal S4 are output as the rectangular wave pulse signals having negative polarity.

As described above, the pulse width of each of the rectangular wave pulse signals S1 to S5 in the pulse group output period P, and the duration of each of the output stop times N1 to N5 are 100 µs. Accordingly, the pulse cycle of each of the rectangular wave pulse signals S1 to S5 in the pulse group output period P is 200 µs, and sufficiently short. Accordingly, the user integrally recognizes these rectangular wave pulse signals S1 to S5 as one electrostimulation. The frequency of each of the rectangular wave pulse signals S1 to S5 in the pulse group output period P is 5,000 Hz.

According to the basic waveforms B1 to B5, no pulse signal is output during the pulse group output suspension periods R1 to R5. The duration of each of the pulse group output suspension periods R1 to R5 is longer than the duration of pulse group output period P. In this embodiment, as shown in Figure 7, the duration of the pulse group output period P is 1 ms. The durations of the pulse group output suspension periods R1 to R5 are 499 ms, 249 ms, 124 ms, 61.5 ms, and 49 ms, respectively. Thus, the pulse group output suspension periods R1 to R5 have a significantly longer duration than the output stop time in the pulse group output period P.

Accordingly, as shown in Figure 7, the first burst wave (2 Hz) includes a pulse group output period P of 1 ms, and a pulse group output suspension period R1 of 499 ms. That is, the first burst wave (2 Hz) is output at a frequency of 2 Hz for the pulse group output period P.

The second burst wave (4 Hz) includes the pulse group output period P of 1 ms, and a pulse group output suspension period R2 of 249 ms. That is, the second burst wave (4 Hz) is output at a frequency of 4 Hz for the pulse group output period P.

The third burst wave (8 Hz) includes the pulse group output period P of 1 ms, and a pulse group output suspension period R3 of 124 ms. That is, the third burst wave (8 Hz) is output at a frequency of 8 Hz for the pulse group output period P.

The fourth burst wave (16 Hz) includes the pulse group output period P of 1 ms, and a pulse group output suspension period R4 of 61.5 ms. That is, the fourth burst wave (16 Hz) is output at a frequency of 16 Hz for the pulse group output period P.

The fifth burst wave (20 Hz) includes the pulse group output period P of 1 ms, and a pulse group output suspension period R5 of 49 ms. That is, the fifth burst wave (20 Hz) is output at a frequency of 20 Hz for the pulse group output period P.

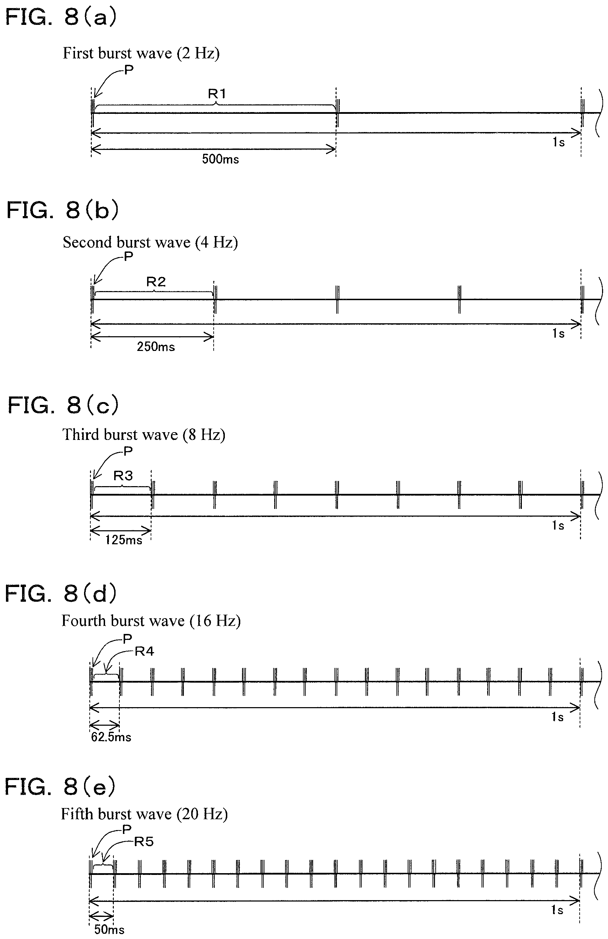

Repetitive output of the basic waveforms B1 to B5 according to a predetermined combination for a predetermined time period allows predetermined burst waves to be output as shown in Figures 8(a) to 8(e). As described above, the user recognizes the multiple rectangular wave pulse signals S1 to S5 in the pulse group output period P as one electrostimulation. Accordingly, as shown in Figure 8(a), electrostimulation at a frequency of 2 Hz is output through the first burst wave repeating the basic waveform B1. Likewise, electrostimulation at a frequency of 4 Hz is output through the second burst wave repeating the basic waveform B2. Electrostimulation at a frequency of 8 Hz is output through the third burst wave repeating the basic waveform B3. Electrostimulation at a frequency of 16 Hz is output through the fourth burst wave repeating the basic waveform B4. Electrostimulation at a frequency of 20 Hz is output through the fifth burst wave repeating the basic waveform B5.

The first to third output modes stored in the output mode memory 405a, i.e., the duration memory, are configured by freely selecting any one of the basic waveforms B1 to B5 stored in the output mode memory 405a and combining burst waves at a predetermined frequency. First, as shown in Table 1, the first output mode is a warm-up mode configured to sequentially perform the following first status to fourth status. The condition of each status is as follows.
(1) In the first status, 100% output is performed through the first burst wave (2 Hz) for 20 seconds. As shown in Figure 9, for five seconds after the start in the first status, what is called a soft start is performed. According to the soft start, the output voltage is gradually increased from 0% to 100%.
(2) In the second status, 100% output is performed through the second burst wave (4 Hz) for 20 seconds.
(3) In the third status, 100% output is performed through the third burst wave (8 Hz) for 10 seconds.
(4) In the fourth status, 100% output is performed through the fourth burst wave (16 Hz) for 10 seconds.

The duration period of the first output mode (i.e., the total of the duration periods of the first status to the fourth status) is one minute. This first output mode is configured so as to gradually increase the frequency of the burst waves from 2 Hz to 16 Hz. Thus, the first output mode is referred to as the warm-up mode. [Table 1]

**(Table 1)**

| First output mode (warm-up mode) | | | | |
|---|---|---|---|---|
| Status | 1 | 2 | 3 | 4 |
| Frequency | 2 Hz | 4 Hz | 8 Hz | 16 Hz |
| Output factor | 100% | 100% | 100% | 100% |
| Output period | 20 sec | 20 sec | 10 sec | 10 sec |
| Duration period | 60 sec (= 1 min) | | | |

Next, as shown in Table 2, the second output mode is a training mode configured to sequentially perform the following first status to fourth status. The condition of each status is as follows.
(1) In the first status, 100% output is performed through the fifth burst wave (20 Hz) for three seconds, and a state without output is maintained for two seconds. This process is repeated for five minutes.
(2) In the second status, 100% output is performed through the fifth burst wave (20 Hz) for three seconds, and subsequently, 100% output is performed through the second burst wave (4 Hz) for two seconds. This process is repeated for five minutes.
(3) In the third status, 100% output is performed through the fifth burst wave (20 Hz) for four seconds, and subsequently, 100% output is performed through the second burst wave (4 Hz) for two seconds. This process is repeated for five minutes.
(4) In the fourth status, 100% output is performed through the fifth burst wave (20 Hz) for five seconds, and subsequently, 100% output is performed through the second burst wave (4 Hz) for two seconds. This process is repeated for five minutes.

As shown in Figure 9, in the second output mode, what is called the soft start is performed. According to the soft start, the output voltage is gradually increased from 0% to 100% for starting five seconds in each of the first status to fourth status.

The duration period of the second output mode is 20 minutes. In this second output mode, the fifth burst wave at the frequency of 20 Hz are maintained for a predetermined time period, and subsequently, a state without output is maintained or the second burst wave at the frequency of 4 Hz is maintained for a predetermined time period. Accordingly, this mode is excellent at effectively stimulating muscles. Thus, the second output mode is referred to as the training mode.

### [Table 2]

**(Table 2)**

| **Second output mode (training mode)** | | | | | | | | |
|---|---|---|---|---|---|---|---|---|
| Status | 1 | | 2 | | 3 | | 4 | |
| Frequency | 20 Hz | No output | 20 Hz | 4 Hz | 20 Hz | 4 Hz | 20 Hz | 4 Hz |
| Output factor | 100% | 0% | 100% | 100% | 100% | 100% | 100% | 100% |
| Output period | 3 sec | 2 sec | 3 sec | 2 sec | 4 sec | 2 sec | 5 sec | 2 sec |
| Duration period | 300 sec (= 5 min) | | 300 sec (= 5 min) | | 300 sec (= 5 min) | | 300 sec (= 5 min) | |

Next, as shown in Table 3, the third output mode is a cool-down mode configured to sequentially perform the following first status to fourth status. The condition of each status is as follows.
(1) In the first status, output is performed through the fourth burst wave (16 Hz) for 10 seconds.
(2) In the second status, output is performed through the third burst wave (8 Hz) for 10 seconds.
(3) In the third status, output is performed through the second burst wave (4 Hz) for 20 seconds.
(4) In the fourth status, output is performed through the first burst wave (2 Hz) for 20 seconds.

As shown in Figure 9, in the third output mode, output in each of statuses is gradually reduced so as to be 100% at the start of the first status and 50% at the end of the fourth status.

The duration period of the third output mode is one minute. In this third output mode, the burst waves are configured to have a frequency gradually decreasing from 16 Hz to 2 Hz. Thus, the third output mode is referred to as the cool-down mode.

### [Table 3]

**(Table 3)**

| **Third output mode (cool-down mode)** | | | | |
|---|---|---|---|---|
| Status | 1 | 2 | 3 | 4 |
| Frequency | 16 Hz | 8 Hz | 4 Hz | 2 Hz |
| Output factor | Gradually reduced from 100% to 50% | | | |
| Output period | 10 sec | 10 sec | 20 sec | 20 sec |
| Duration period | 60 sec (= 1 min) | | | |

As described above, the total time of sequential execution of the first output mode (warm-up mode), the second output mode (training mode), and the third output mode (cool-down mode) is 22 minutes. In this embodiment, as shown in Figure 9, pause periods each having two seconds are provided between the first output mode and the second output mode, and four points between the statuses in the second output mode. Accordingly, the total time of the entire process including the pause periods is 22 minutes and 8 seconds.

Next, a usage mode in the muscle electrostimulation device 1 of this embodiment is described in detail.

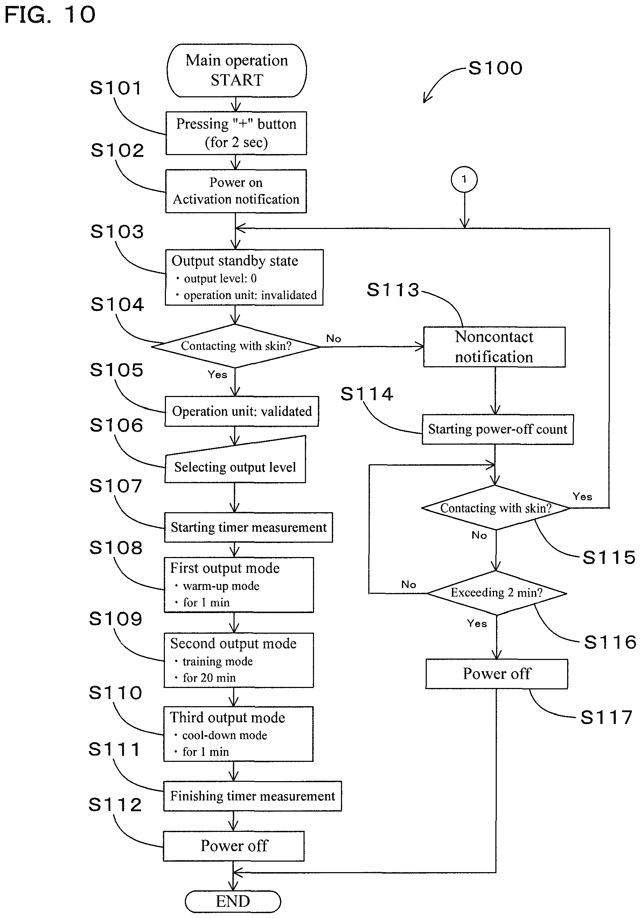

A main operation flow S100 shown in Figure 10 is described. In the main operation flow S100, first, "+" on the operation surface 54 is pressed for two seconds (S101). Accordingly, the power of the muscle electrostimulation device 1 is turned on, the muscle electrostimulation device 1 is activated, and a notification sound ("beep") for notification of activation is made by the loudspeaker 43 (S102). Subsequently, the muscle electrostimulation device 1 is brought into an output standby state, the output level is set to zero, and input into the operation unit 50 is invalidated (S103).

Next, the skin detector 402 detects whether the electrode unit 30 is in contact with skin or not (S104). If the skin detector 402 detects that the electrode unit 30 is in contact with the skin (Yes in S104), the operation unit 50 is validated (S105). The output level is then input through the operation unit 50 (S106). The output level is input onto the operation surface 54 of the operation unit 50. Every press of "+" of the operation surface 54 of the operation unit 50 increments the output level by one level. Every press of "-" of the operation surface 54 decrements the output level by one level. Upon setting of the output level, the output start signal is transmitted from the controller 40 to the timer 404, and measurement is started at the timer 404 (S107). The operation of the output level can be allowed at any point in usage time (from activation of the operation unit 50 to power off).

From start of measurement at the timer 404 (elapsed time of 0) to an elapsed time of one minute, the output mode in the electrode unit 30 is set to the first output mode (warm-up mode) (S108). When the elapsed time reaches one minute, the output mode switcher 405 as the frequency setting unit switches the output mode in the electrode unit 30 to the second output mode (training mode), and maintains this mode until reaching an elapsed time of 21 minutes, i.e., for 20 minutes (S109). When the elapsed time reaches 21 minutes, the output mode switcher 405 as the frequency setting unit switches the output mode in the electrode unit 30 to the third output mode (cool-down mode), and maintains this mode until reaching an elapsed time 22 minutes, i.e., for one minute (S110). When the elapsed time reaches 22 minutes, measurement at the timer 404 is finished (S111). The muscle electrostimulation device 1 is then stopped (S112). Such execution of steps S108 to S111 thus performs one set of the first output mode (warm-up mode), the second output mode (training mode) and the third output mode (cool-down mode), and the process is finished.

On the other hand, if the skin detector 402 determines that the electrode unit 30 is not in contact with the skin (No in S104), a notification sound ("beep, beep, beep") for notification of the noncontact state is made by the loudspeaker 43 (S113). The count start signal is transmitted from the controller 40 to the power-off counter 403, and the power-off counter 403 starts measurement of the elapsed time (S114).

Next, the skin detector 402 detects whether the electrode unit 30 is in contact with the skin or not (S115). If the skin detector 402 detects that the electrode unit 30 is in contact with the skin, the processing returns to step S103 described above, and the state is brought into the output standby state (Yes in S115). On the contrary, if the skin detector 402 detects that the electrode unit 30 is not in contact with the skin (No in S115), it is determined whether the elapsed time at the power-off counter 403 exceeds two minutes or not (S116). If it is determined that the elapsed time at the power-off counter 403 does not exceed two minutes (No in S116), the processing returns to S115 again, and the skin detector 402 detects whether the electrode unit 30 is in contact with the skin or not. On the contrary, if it is determined in S116 that the elapsed time at the power-off counter 403 exceeds two minutes (Yes in S116), the power of the muscle electrostimulation device 1 is turned off (S117).

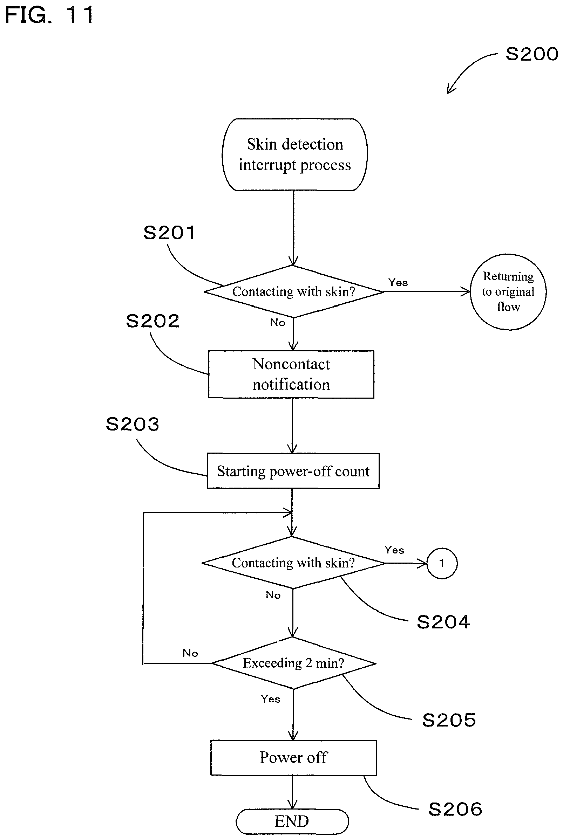

Next, an interrupt process to interrupt and be preferentially processed between S105 to S110 in the aforementioned main operation flow S100 is described. As shown in Figure 11, a skin detection interrupt process S200 is performed as a first interrupt process. The skin detection interrupt process S200 is used as an automatic power-off function in case where the electrodes are apart from the human body in the middle of usage. In the skin detection interrupt process S200, first, the skin detector 402 detects whether the electrode unit 30 is in contact with the skin or not (S201). If the skin detector 402 detects that the electrode unit 30 is in contact with the skin (Yes in S201), the processing returns to the original flow of the main operation flow S100. On the contrary, if the skin detector 402 determines that the electrode unit 30 is not in contact with the skin (No in S201), a notification sound ("beep, beep, beep") for notification of the noncontact state is made by the loudspeaker 43 (S202). The count start signal is transmitted from the controller 40 to the power-off counter 403, and the power-off counter 403 starts measurement of the elapsed time (S203).

Next, the skin detector 402 detects whether the electrode unit 30 is in contact with the skin or not (S204). If the skin detector 402 detects that the electrode unit 30 is in contact with the skin, the processing returns to step S103 in the main operation flow S100 (Yes in S204). On the contrary, if the skin detector 402 determines that the electrode unit 30 is not in contact with the skin (No in S204), it is determined whether the elapsed time at the power-off counter 403 exceeds two minutes or not (S205). If it is determined that the elapsed time at the power-off counter 403 does not exceed two minutes (No in S205), the processing returns to S204 again, and the skin detector 402 detects whether the electrode unit 30 is in contact with the skin or not. On the contrary, if it is determined in S205 that the elapsed time at the power-off counter 403 exceeds two minutes (Yes in S205), the power of the muscle electrostimulation device 1 is turned off (S206).

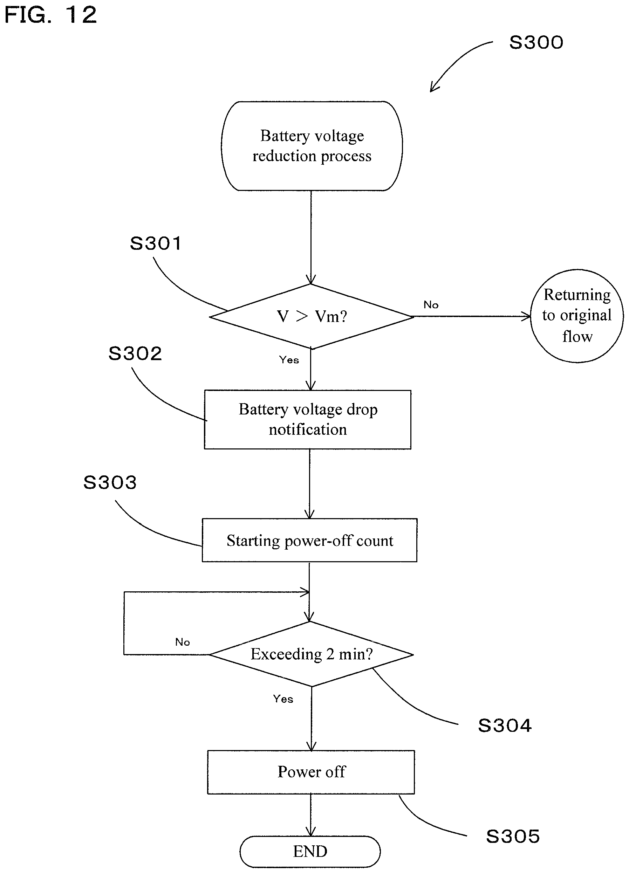

Next, as shown in Figure 12, a battery voltage reduction process S300 is described. This process is a second interrupt process to interrupt and be preferentially processed between steps S105 to S110 in the main operation flow S100, described above. The battery voltage reduction process S300 is a function of automatic power-off in case where the battery voltage of the battery 21 drops. Accordingly, the user can easily notice the case where the battery is required to be replaced and the like. First, the battery voltage detector 406 determines whether the detected battery voltage V of the battery 21 in the power source 20 is lower than the predetermined threshold Vm or not (S301). If it is determined that the battery voltage V is not lower than the predetermined threshold Vm (No in S301), the processing returns to the original flow of the main operation flow S100. On the contrary, if it is determined that the battery voltage V is lower than the predetermined threshold Vm, the notification sound ("beep, beep, beep") for notification of this determination is made by the loudspeaker 43 (S302). The count start signal is transmitted from the controller 40 to the power-off counter 403, and the power-off counter 403 starts measurement of the elapsed time (S303).

Next, it is determined whether the elapsed time at the power-off counter 403 exceeds two minutes or not (S304). If it is determined that the elapsed time at the power-off counter 403 does not exceed two minutes (No in S304), the processing returns to S304 again. If it is determined that the elapsed time at the power-off counter 403 exceeds two minutes (Yes in S304), the power of the muscle electrostimulation device 1 is turned off (S305).

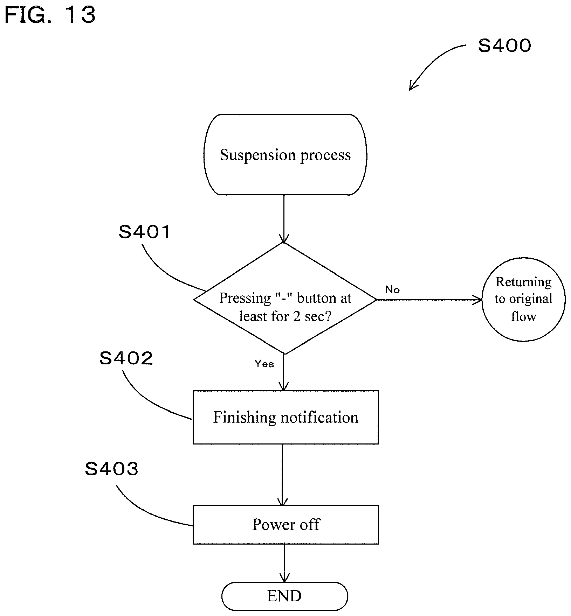

Next, as shown in Figure 13, a suspension process S400 is described. This process is a third interrupt process to interrupt and be preferentially processed between S105 to S110 in the main operation flow S100, described above. First, the controller 50 determines whether the time of pressing the "-" button on the operation surface 54 of the operation unit 50 is at least two seconds or not (S401). If it is determined that the time of pressing the "-" button is not at least two seconds (No in S401), the processing returns to the original flow of the main operation flow S100. On the contrary, if it is determined that the time of pressing the "-" button is at least two seconds (Yes in S401), a notification sound ("beep") for notification that the power of the muscle electrostimulation device 1 is turned off and the process is finished is made by the loudspeaker 43 (S402). The power is then turned off (S403).

The effects of muscle electrostimulation device 1 of this embodiment are hereinafter described.

In the muscle electrostimulation device 1 of this embodiment, through the first to fifth burst waves for forming electrostimulation, the rectangular wave pulse signals S1 to S5 are output, with the output stop times N1 to N5 being inserted between the rectangular wave pulse signals S1 to S5, in the pulse group output period P. Thus, in the pulse group output period P, the rectangular wave pulse signals S1 to S5 are in a state of being divided into multiple pieces. Accordingly, in comparison with the case of continuously outputting the rectangular wave pulse signals S1 to S5 without division over the pulse group output period P, each of individual pulse widths of the rectangular wave pulse signals S1 to S5 can be small while maintaining the same total output time of rectangular wave pulse signals S1 to S5. As a result, the pain of the user can be alleviated while maintaining electrostimulation being outputted from the muscle electrostimulation device 1 and flowing into muscles or nerves connected to muscles. Accordingly, the sensitivity during use of the muscle electrostimulation device 1 can be improved.

The burst waves (basic waveforms B1 to B5) are configured such that the rectangular wave pulse signals S1 to S5 are output, with the output stop times N1 to N5 being inserted between the rectangular wave pulse signals S1 to S5, in the pulse group output period P. These bust waves have the same period P of outputting pulses as burst waves having a pulse outputting period continuously outputting pulses for a period identical to the pulse group output period P. Accordingly, the burst waves having the pulse group output periods P, with the output stop times N1 to N5 being inserted between the rectangular wave pulse signals S1 to S5, can achieve sensitivity similar to the sensitivity of the burst wave having a pulse outputting period with no output stop time being inserted between the rectangular wave pulse signals.

In the pulse group output period P, the rectangular wave pulse signals S1 to S5 are output, with the output stop times N1 to N5 being inserted between the rectangular wave pulse signals S1 to S5. Thus, the duration of the pulse group output period P is the total of the pulse widths of the rectangular wave pulse signals S1 to S5 and all the output stop times N1 to N5. Accordingly, in comparison with the case of outputting the rectangular wave pulse signals S1 to S5 without division over the duration of the pulse group output period P, the actual pulse signal output time is reduced by the output stop times N1 to N5 while maintaining the duration of the pulse group output period P to be the same. The power consumption can therefore be reduced. This reduction allows driving even by a power source having a small capacity, thereby contributing to reduction in size of the device.

The burst wave forming electrostimulation includes the pulse group output period P and any of the pulse group output suspension periods R1 to R5. The durations of the pulse group output suspension periods R1 to R5 are longer than the output stop times N1 to N5 in the pulse group output period P. Since the burst waves include such pulse group output suspension periods R1 to R5, the frequencies of the burst waves can be set to a desired value by only changing the duration of each of the pulse group output suspension periods R1 to R5 to a predetermined length without changing the pulse group output period P. This setting facilitates control for outputting electrostimulation including the burst wave having a frequency suitable for contracting and relaxing muscles, and can efficiently stimulate the muscles.

The repetitive burst waves (B1 to B5) include the rectangular wave pulse signals S1, S3 and S5 having positive polarity, and the rectangular wave pulse signals S2 and S4 having negative polarity. Accordingly, deviation in electric charge can be reduced. The reduction can alleviate the pain of the user. As a result, the sensitivity and usefulness during use of the muscle electrostimulation device 1 can be improved.

In this embodiment, the rectangular wave pulse signals S1, S3 and S5 having positive polarity, and the rectangular wave pulse signals S2 and S4 having negative polarity are included in the same pulse group output period P. Accordingly, deviation in electric charge can be easily eliminated in each burst wave (basic waveforms B1 to B5), thereby allowing the pain of the user to be further alleviated. As a result, the sensitivity and usefulness during use of the muscle electrostimulation device 1 can be further improved.

Furthermore, in the case of outputting the five rectangular wave pulse signals S1 to S5 output in the first pulse group output period P of the first burst wave in an order of "positive, negative, positive, negative, and positive" as in this embodiment, the five rectangular wave pulse signals output in the second pulse group output period of the second burst wave occurring subsequent to the first burst wave can be output in an order of "negative, positive, negative, positive, and negative". In this case, the deviation in electric charge having occurred in the first burst wave can be securely eliminated by the second burst wave. Accordingly, the pain of the user can be further alleviated. Furthermore, the second pulse group output period can be generated only by inverting the polarities of the rectangular wave pulse signals S1 to S5 output in the first pulse group output period P (inverting the potential). Accordingly, the control load can be reduced in comparison with the case of separately controlling the polarities of the individual rectangular wave pulse signals in each pulse group output period.

In this embodiment, the rectangular wave pulse signals S1, S3 and S5 having positive polarity, and the rectangular wave pulse signals S2 and S4 having negative polarity are included in the same pulse group output period P. In multiple burst waves, the first burst wave may include rectangular wave pulse signals having positive polarity, and the second burst wave different from the first burst wave may include rectangular wave pulse signals having negative polarity. For example, all the rectangular wave pulse signals S1 to S5 in the first pulse group output period P of the first burst wave may have the positive polarity, all the rectangular wave pulse signals in the second pulse group output period of the second burst wave subsequent to the first burst wave with intervention of any of the pulse group output suspension periods R1 to R5 may have the negative polarity, and the first burst wave and the second burst wave may be repeated. In this case, in the individual pulse group output periods, the rectangular wave pulse signals have the same polarities. However, the entire burst waves repeatedly output include rectangular wave pulse signals having polarities different from each other. Also in such a case, the deviation in electric charge having occurred in the first burst wave can be securely eliminated by the second burst wave. Accordingly, the pain of the user can be further alleviated.

In this embodiment, each of the durations of the pulse group output suspension periods R1 to R5 is longer than the duration of the pulse group output period P (1 ms). Accordingly, the pulse group output suspension periods R1 to R5 sufficiently secure the interval between pulse group output periods P repeatedly output through the burst wave. The secured interval allows the user to easily recognize the multiple rectangular wave pulse signals S1 to S5 in the pulse group output period P as one electrostimulation. As a result, the low frequency (2 to 20 Hz in this embodiment) burst waves can be easily output from the rectangular wave pulse signals S1 to S5 having high frequency (frequency of 5,000 Hz in this embodiment), thereby allowing electrostimulation suitable for stimulating muscles to be output.

This embodiment includes: the burst wave pattern memory (output mode memory 405a) preliminarily storing the multiple burst wave patterns (basic waveforms B1 to B5) having different frequencies from each other due to difference in duration of the pulse group output suspension periods R1 to R5, while the duration of the pulse group output period P being the same; and the frequency setting unit (output mode switcher 405) for setting the frequency of the burst wave for electrostimulation by selecting any of the burst wave patterns (basic waveforms B1 to B5) stored in the burst wave pattern memory (output mode memory 405a). Thus, since the burst wave pattern memory (output mode memory 405a) preliminarily stores the multiple burst wave patterns (basic waveforms B1 to B5) having predetermined frequencies, changing of the frequency of the burst wave only requires the frequency setting unit (output mode switcher 405) to select a predetermined pattern from among the burst wave patterns stored in the burst wave pattern memory (output mode memory 405a). This requirement facilitates changing of the frequency of the burst wave. Accordingly, the muscle electrostimulation device 1 suitable for efficiently stimulating muscles can be achieved.

In this embodiment, the pulse width of the rectangular wave pulse signals S1 to S5 and the output stop times N1 to N5 in the burst wave are constant. Accordingly, the electrostimulation to be applied to muscles can be easily changed on the basis of the frequency of the burst wave. Therefore, adjustment of the electrostimulation by the frequency of the burst wave becomes easier, thereby facilitating output of electrostimulation suitable for effectively stimulating the muscles.

This embodiment includes: the main body 10; multiple electrode units 30 for outputting electrostimulation; the power source 20 for supplying power to the electrode unit 30; the controller 40 for controlling power supply by the power source 20; and the operation unit 50 configured to be capable of changing the control mode of the controller 40. The power source 20 is embedded in the main body 10. This configuration does not need to externally prepare electric power to be supplied to the electrode unit 30. Accordingly, the device can be easily used even outdoors and in places away from home where it is difficult to secure a power source. Furthermore, this configuration does not require a cord and the like for connection to the power source. Accordingly, the usability is improved, and the portability is excellent.

In this embodiment, the electrode unit 30 includes the multiple electrodes 311 to 313 and 321 to 323, and the leads 311a to 313a and 321a to 323a for electrically connecting the electrodes 311 to 313 and 321 to 323 to the power source 30 via the controller 40, on the sheet-shaped substrate 33 extending from the main body 10. Accordingly, the electrode unit 30 is formed on the sheet-shaped substrate 33 extending from the main body 10, thereby allowing the main body 10 and the electrode unit 30 to be integrally configured. This configuration does not require a cord and the like for connecting the main body 10 to the electrode unit 30. Accordingly, the usability is improved, and the portability is excellent.

In this embodiment, the electrode unit 30 includes at least three electrodes 311 to 313 and 321 to 323. As described above, since the pulse group output period P includes the output stop times N1 to N5, the power consumption is reduced. Accordingly, even the configuration including at least three electrodes 311 to 313 and 321 to 323 can apply sufficient electrostimulation. Therefore, electrostimulation can be applied to a wide range of muscles, thereby allowing muscles to be efficiently stimulated.

In this embodiment, the power source 20 includes the replaceable battery 21. Accordingly, power is supplied only by replacing the battery 21, thereby facilitating long-time use exceeding the capacity of the battery. This configuration does not require embedding a battery having an excessively large capacity, thereby allowing the device to be reduced in size.

In this embodiment, the battery 21 is a coin battery. Accordingly, the battery 21 has a small size, thereby contributing to reduction in size of the muscle electrostimulation device 1. The reduction in size of the muscle electrostimulation device 1 can, in turn, reduce the weight. Accordingly, the electrode unit 30 becomes resistant to falling and dropping off the body of the user, thereby improving the usability and portability. Furthermore, the battery 21 is thin, thereby contributing to reduction in thickness of the muscle electrostimulation device 1. The thinly shaped muscle electrostimulation device 1 allows the user to wear clothes on muscle electrostimulation device 1, with the device being left to be worn. Accordingly, the muscle electrostimulation device 1 can be used during commuting to the workplace or school, operations of household and work, and in other various situations. The coin battery has higher operating voltage and more stable discharge characteristics than the other dry batteries. Accordingly, the muscle electrostimulation device 1 can be stably operated for a relatively long time.

The battery 21 may be a battery having a nominal voltage ranging from 3.0 to 5.0 V. This embodiment adopts the battery 21 having a nominal voltage of 3.0 V. The electronic component 42, the loudspeaker 43 and the like included in the muscle electrostimulation device 1 have the same drive voltage. Accordingly, a step-down circuit or a booster circuit is not required to be separately provided to drive these electronic components 42 and 43, thereby contributing to reduction in size.

The power source 20 is embedded with a rechargeable battery instead of the replaceable battery 21. A power supply terminal connectable to an external power source may be provided as means for charging such a battery. Alternatively, noncontact power supply unit using electromagnetic induction may be provided. In this case, the battery can be repeatedly used. Accordingly, this case can reduce expendable supplies in comparison with the case of using nonchargeable battery.

As described above, this embodiment can provide the muscle electrostimulation device 1 capable of improving sensitivity in use and efficiently stimulating muscles.

In this embodiment, the second output mode (training mode) is executed on the basis of the first status to fourth status shown in Table 2, described above. Alternatively, as with the following Variation 1, in a first status to a fourth status equivalent to the statuses of this embodiment, a 2a status shown in Table 4 may be executed between the second status and the third status, and a 3a status shwon in Table 4 may be executed between the third status and the fourth status.

**(Table 4)**

| **Second output mode (training mode)** | | | | | | | | | | | | | | |
|---|---|---|---|---|---|---|---|---|---|---|---|---|---|---|
| Status | 1 | | 2 | | 2a | | | 3 | | 3a | | | 4 | |
| Frequency | 20 Hz | No output | 20 Hz | 4 Hz | 4 Hz | 8 Hz | 16 Hz | 20 Hz | 4 Hz | 4 Hz | 8 Hz | 16 Hz | 20 Hz | 4 Hz |
| Output factor | 100% | 0% | 100% | 100% | 100% | 100% | 100% | 100% | 100% | 100% | 100% | 100% | 100% | 100% |
| Output period | 3 sec | 2 sec | 3 sec | 2 sec | 10 sec | 10 sec | 10 sec | 4 sec | 2 sec | 10 sec | 10 sec | 10 sec | 5 sec | 2 sec |
| Duration period | 300 sec (= 5 min) | | 300 sec (= 5 min) | | 30 sec (= 0.5 min) | | | 300 sec (= 5 min) | | 30 sec (= 0.5 min) | | | 300 sec (= 5 min) | |

In Variation 1, as shown in Table 4, the 2a status and the 3a status are executed as follows.
(2a) In the 2a status, 100% output is performed through the second burst wave (4 Hz) for 10 seconds, then 100% output is performed through the third burst wave (8 Hz) for 10 seconds, and subsequently, 100% output is performed through the fourth burst wave (16 Hz) for 10 seconds.
(3a) In the 3a status, 100% output is performed through the second burst wave (4 Hz) for 10 seconds, then 100% output is performed through the third burst wave (8 Hz) for 10 seconds, and subsequently, 100% output is performed through the fourth burst wave (16 Hz) for 10 seconds.

In this Variation 1, the 2a status and the 3a status are added, in comparison with the second output mode of this embodiment (see Table 2). Accordingly, the total time of sequential execution of the first output mode (warm-up mode), the second output mode (training mode) and the third output mode (cool-down mode) shown in Table 4 is 23 minutes.

In the 2a status, the frequency of the burst wave is configured to be stepwisely increased from 4 Hz to 16 Hz. Accordingly, variation in frequency in the case of switching from the 2a status to the third status is smoothed. Likewise, variation in frequency in the case of switching from the 3a status to the fourth status is smoothed. In this Variation 1, the 2a status and the 3a status are added in comparison with the case of Embodiment 1, thereby largely changing the pattern of electrostimulation in the second output mode (training mode). As a result, the change in pattern can prevent reduction in sensitivity due to user's getting used to the electrostimulation, thereby allowing the rectus abdominis muscles to be more effectively stimulated. Furthermore, adoption of the 2a status and the 3a status exerts an advantageous effect of sweeping away fatigue substances in muscles fatigued by application of electrostimulation. This Variation 1 with the second output mode (training mode) being set as such also exerts effects equivalent to the effects of Embodiment 1.

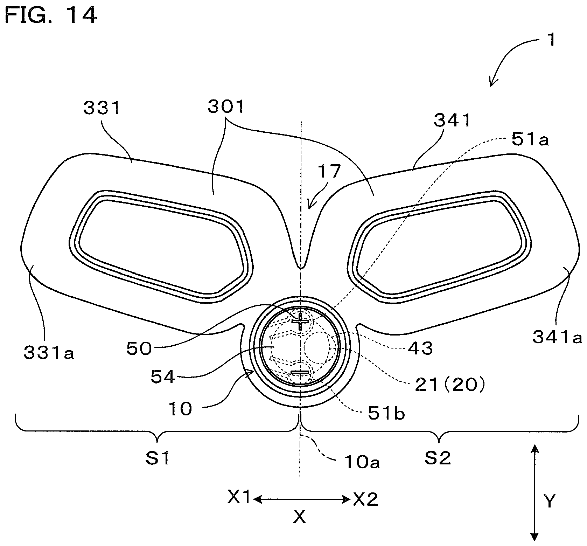

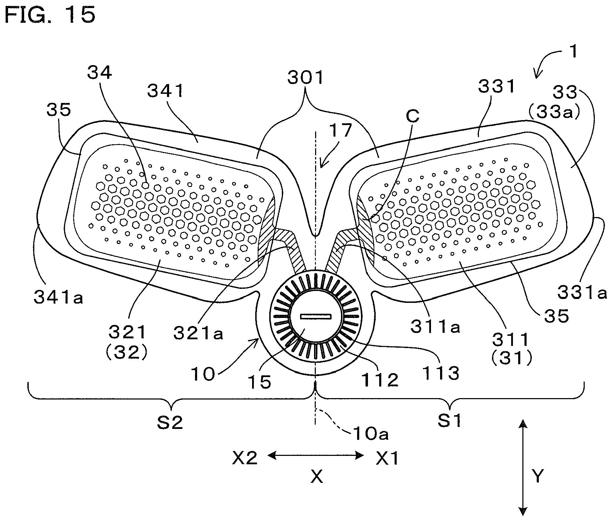

Embodiment 1 thus includes the six electrodes 311 to 313 and 321 to 323. The configuration is not limited to this embodiment. Alternatively, the number of electrodes may be two or more. For example, in Variation 2, as shown in Figures 14 and 15, electrodes have a configuration similar to the configuration of the electrodes 311 and 321 in Embodiment 1. However, a pair of one-size-larger electrodes 311 and 321 is provided. In Variation 2, the same symbols are assigned to configuration elements equivalent to the elements in Embodiment 1, and the description is omitted. This case also exerts effects equivalent to the effects of Embodiment 1. The muscle electrostimulation device 1 of Variation 2 has a smaller number of electrodes 311 and 321 than the case of having six electrodes (see Figure 2). Accordingly, the power consumption per electrode can be set high. Thus, each of the electrodes 311 and 321 is one-size larger. Accordingly, the range of capable of applying electrostimulation per one electrode is increased, thereby facilitating stimulation of muscles of large parts, such as arms and thighs.

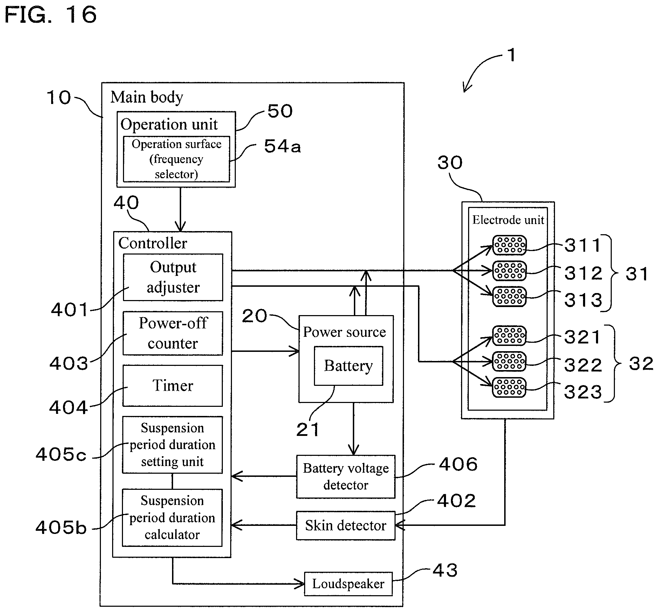

In Embodiment 1, for changing the frequency of the burst wave, the frequency setting unit (output mode switcher 405) selects a predetermined one from among the burst wave patterns stored in the burst wave pattern memory (output mode memory 405a). Alternatively, the following Variation 3 may be adopted. As shown in Figure 16, Variation 3 includes: an operation surface 54a as a frequency selector for selecting the frequency of the burst wave; a suspension period duration calculator 405b; and a suspension period duration setting unit 405c.

The suspension period duration calculator 405b calculates the duration of the pulse group output suspension period on the basis of the frequency selected by the frequency selector (operation surface 54a).

The suspension period duration setting unit 405c sets the duration of the pulse group output suspension period on the basis of the duration calculated by the suspension period duration calculator 405b.

In Variation 3, the same symbols are assigned to configuration elements equivalent to the elements in Embodiment 1, and the description is omitted.

According to such Variation 3, the frequency selector (operation surface 54a) can set the frequency of the burst wave to a desired frequency. Accordingly, the frequency of the burst wave is appropriately set on the basis of the preferences of users (the strength of contraction, and the interval between contraction and relaxation), thereby allowing the muscle electrostimulation device 1 to further efficiently stimulate muscles in conformity with the individual users. This Variation 3 also exerts effects equivalent to the effects of Embodiment 1, except for the effects pertaining to the modes of changing the frequency of the burst wave.

The present invention is set out in the appended claims. The embodiments, aspects or examples according to the present description that do not fall within the scope of said claims are provided for illustrative purposes only and do not form part of the present invention.

## Claims

1. A muscle electrostimulation device (1) configured to apply electrostimulation to a muscle,
wherein the electrostimulation includes repeatedly output multiple burst waves, the burst waves each including a pulse group output period for outputting multiple rectangular wave pulse signals, with an output stop time being inserted between the rectangular wave pulse signals, and a pulse group output suspension period for suspending output of the rectangular wave pulse signals, the pulse group output suspension period being longer than the output stop time, and
the repeated burst waves each include at least one rectangular wave pulse signal having positive polarity and at least one rectangular wave pulse signal having negative polarity,
the muscle electrostimulation device comprising:
a main body (10);
an electrode unit (30) for outputting the electrostimulation;
a power source (20) for supplying power to the electrode unit, the power source being embedded in the main body;
a controller (40) for controlling power supply by the power source; and
an operation unit (50) configured to be capable of changing a control mode of the controller,
wherein the electrode unit includes multiple electrodes (311-313, 321-323) and leads for electrically connecting the electrodes to the power source via the controller, and the electrodes and the leads are formed on a sheet-shaped substrate (33) extending from the main body.

2. The muscle electrostimulation device according to claim 1, wherein a duration of the pulse group output suspension period is longer than a duration of the pulse group output period.

3. The muscle electrostimulation device according to claim 2, comprising:
a burst wave pattern memory (405a) for preliminarily storing multiple burst wave patterns different in frequency due to a difference in the duration of the pulse group output suspension period while being identical in the duration of the pulse group output period; and
a frequency setting unit (405) for setting a frequency of each of the burst waves for the electrostimulation by selecting any of the burst wave patterns stored in the burst wave pattern memory.

4. The muscle electrostimulation device according to claim 1, wherein the electrode unit (30) includes at least three electrodes.

5. The muscle electrostimulation device according to any one of claims 1 and 4, wherein the power source (20) includes a replaceable battery (21).

6. The muscle electrostimulation device according to claim 5, wherein the battery (21) is a coin battery or a button battery.

## Patentansprüche

1. Muskelelektrostimulationsvorrichtung (1), die zum elektrisch Stimulieren eines Muskels ausgebildet ist,
bei der die elektrische Stimulation ein wiederholtes Ausgeben mehrerer Burst-Wellen beinhaltet, die jeweils eine Pulsgruppenausgabeperiode zum Ausgeben mehrerer Rechteckwellenpulssignale, wobei eine Ausgabestoppzeit zwischen die Rechteckwellenpulssignale eingefügt ist, und eine Pulsgruppenausgabeunterbrechungsperiode zum Unterbrechen der Ausgabe der Rechteckwellenpulssignale, wobei die Pulsgruppenausgabeunterbrechungsperiode länger als die Ausgabestoppzeit ist, aufweisen und
die wiederholten Burst-Wellen jeweils mindestens ein Rechteckwellenpulssignal mit positiver Polarität und mindestens ein Rechteckwellenpulssignal mit negativer Polarität aufweisen,
bei der die Muskelelektrostimulationsvorrichtung aufweist:
einen Hauptkörper (10);
eine Elektrodeneinheit (30) zum Ausgeben der Elektrostimulation;
eine Leistungsquelle (20) zum Zuführen von Leistung zu der Elektrodeneinheit, wobei die Leistungsquelle in den Hauptkörper eingebettet ist;
eine Steuerung (40) zum Steuern der Leistungszufuhr durch die Leistungsquelle; und
eine Bedieneinheit (50), die zum Ändern eines Steuermodus der Steuerung ausgebildet ist,
bei der die Elektrodeneinheit mehrere Elektroden (311-313, 321-323) und Leitungen zum elektrischen Verbinden der Elektroden mit der Leistungsquelle über die Steuerung aufweist und die Elektroden und die Leitungen auf einem plattenförmigen Substrat (33) ausgebildet sind, das sich von dem Hauptkörper erstreckt.

2. Muskelelektrostimulationsvorrichtung nach Anspruch 1, bei der eine Dauer der Pulsgruppenausgabeunterbrechungsperiode länger als eine Dauer der Pulsgruppenausgabeperiode ist.

3. Muskelelektrostimulationsvorrichtung nach Anspruch 2, mit:
einem Burst-Wellenmusterspeicher (405a) zum vorläufig Speichern mehrerer Burst-Wellenmuster, die sich aufgrund eines Unterschieds der Dauer der Pulsgruppenausgabeunterbrechungsperiode hinsichtlich der Frequenz unterscheiden, während sie hinsichtlich der Dauer der Pulsgruppenausgabeperiode identisch sind; und
einer Frequenzeinstelleinheit (405) zum Einstellen einer Frequenz jeder der Burst-Wellen für die Elektrostimulation durch Auswählen eines der in dem Burst-Wellenmusterspeicher gespeicherten Burst-Wellenmuster.

4. Muskelelektrostimulationsvorrichtung nach Anspruch 1, bei der die Elektrodeneinheit (30) mindestens drei Elektroden aufweist.

5. Muskelelektrostimulationsvorrichtung nach einem der Ansprüche 1 und 4, bei der die Leistungsquelle (20) eine austauschbare Batterie (21) aufweist.

6. Muskelelektrostimulationsvorrichtung nach Anspruch 5, bei der die Batterie (21) eine münzförmige Batterie oder eine Knopfzelle ist.

## Revendications

1. Dispositif d'électrostimulation musculaire (1) configuré pour appliquer l'électrostimulation à un muscle,
dans lequel l'électrostimulation comprend des salves multiples à sortie répétée, les salves comprenant chacune une période de sortie de train d'impulsions pour délivrer plusieurs signaux d'impulsions d'ondes rectangulaires, un temps d'arrêt de sortie étant inséré entre les signaux d'impulsions d'ondes rectangulaires, et une période de suspension de sortie de train d'impulsions pour suspendre la sortie des signaux d'impulsions d'ondes rectangulaires, la période de suspension de sortie de train d'impulsions étant supérieure à celle du temps d'arrêt de sortie, et
les salves répétées comprennent chacune au moins un signal d'impulsion d'ondes rectangulaires ayant une polarité positive et au moins un signal d'impulsion d'ondes rectangulaires ayant une polarité négative,
le dispositif d'électrostimulation musculaire comprenant :
un corps principal (10) ;
une unité d'électrodes (30) pour délivrer l'électrostimulation ;
une source d'énergie (20) pour fournir de l'énergie à l'unité d'électrodes, la source d'énergie étant intégrée dans le corps principal ;
un contrôleur (40) pour commander l'alimentation électrique par la source d'énergie ; et
une unité de commande (50) configurée pour pouvoir changer un mode de commande du contrôleur,
dans lequel l'unité d'électrodes comprend plusieurs électrodes (311-313, 321-323) et des conducteurs pour connecter électriquement les électrodes à la source d'énergie via le contrôleur, et les électrodes et les conducteurs sont formés sur un substrat en forme de feuille (33) s'étendant du corps principal.

2. Dispositif d'électrostimulation musculaire selon la revendication 1, dans lequel une durée de la période de suspension de sortie de train d'impulsions est supérieure à une durée de la période de sortie de train d'impulsions.

3. Dispositif d'électrostimulation musculaire selon la revendication 2, comprenant :
une mémoire (405a) de motifs de salves pour stocker préalablement plusieurs motifs de salves de fréquences différentes en raison d'une différence dans la durée de la période de suspension de sortie de train d'impulsions tout en étant identiques dans la durée de la période de sortie de train d'impulsions ; et
une unité de réglage de fréquence (405) pour régler une fréquence de chacune des salves pour l'électrostimulation en sélectionnant l'un quelconque des motifs de salves stockés dans la mémoire de motifs de salves.

4. Dispositif d'électrostimulation musculaire selon la revendication 1, dans lequel l'unité d'électrodes (30) comprend au moins trois électrodes.

5. Dispositif d'électrostimulation musculaire selon l'une quelconque des revendications 1 et 4, dans lequel la source d'énergie (20) comprend une batterie remplaçable (21).

6. Dispositif d'électrostimulation musculaire selon la revendication 5, dans lequel la batterie (21) est une pile type pièce de monnaie ou une pile bouton.
